(19) Europäisches Patentamt
European Patent Office
Office européen des brevets

(11) Veröffentlichungsnummer: **0 474 213 B1**

(12) **EUROPÄISCHE PATENTSCHRIFT**

(45) Veröffentlichungstag der Patentschrift: **20.09.95**

(51) Int. Cl.⁶: **C07K 14/625**, C07K 1/20

(21) Anmeldenummer: **91114936.7**

(22) Anmeldetag: **04.09.91**

(54) **Verfahren zur chromatographischen Reinigung von Insulinen.**

(30) Priorität: **05.09.90 DE 4028120**

(43) Veröffentlichungstag der Anmeldung:
**11.03.92 Patentblatt 92/11**

(45) Bekanntmachung des Hinweises auf die
Patenterteilung:
**20.09.95 Patentblatt 95/38**

(84) Benannte Vertragsstaaten:
**AT BE CH DE DK ES FR GB GR IT LI LU NL SE**

(56) Entgegenhaltungen:
DE-A- 3 147 842
GB-A- 2 119 248

CHEMICAL ABSTRACTS, Band 88, Nr. 24, 12.
Juni 1978, Columbus, Ohio, USA
L.K.SHATAEVA et al. "Thermodynamics of
poly- functional interactions during ionicexchange" Seite 475, linke Spalte, Zusammen-
fassung-Nr. 177 670m

(73) Patentinhaber: **HOECHST AKTIENGESELL-SCHAFT**

**D-65926 Frankfurt am Main (DE)**

(72) Erfinder: **Dickhardt, Rainer, Dr.**
**Hallwielweg 2a**
**W-6233 Kelkheim/Taunus (DE)**
Erfinder: **Unger, Bernhard, Dr.**
**Kelkheimer Strasse 12**
**W-6233 Kelkheim/Taunus (DE)**
Erfinder: **Häfner, Leonhard, Dr.**
**Am Erdbeerstein 26**
**W-6240 Königstein/Taunus (DE)**

**Beschreibung**

Die Erfindung betrifft ein Verfahren zur Reinigung von Insulin und/oder Insulinderivaten durch Chromatographie in wäßrigen, gepufferten Lösungsmitteln, die mit Wasser mischbare organische Lösungsmittel enthalten, an lipophil modifiziertem Kieselgel, wobei in dem wäßrigen, gepufferten Lösungsmittel Zwitterionen gelöst sind und/oder der pH-Wert des Lösungsmittels in der Nähe des isoelektrischen Punkts des zu reinigenden Insulins oder Insulinderivats liegt.

Aus analytischen Trennverfahren ist die Reinigung von Insulinen oder Insulinderivaten an lipophil modifizierten (reversed phase) Kieselgelen bekannt und wird seit vielen Jahren in der Hochdruckflüssigkeitschromatographie (HPLC) erfolgreich angewendet (WS Welinder et al., J. Chrom., 361, (1986) 357-367). Im analytischen Maßstab werden Proteinmengen im $\mu$g-Bereich auf eine mit modifiziertem Kieselgel gefüllte Säule aus Stahl, Glas oder Kunststoff aufgetragen und anschließend durch ein strömendes Flüssigkeitsgemisch (zumeist saure, wäßrige, Pufferlösungen mit konstanter oder variabler organischer Lösemittelkonzentration) eluiert. Die Proteinbeladung beträgt hierbei weit weniger als 30 $\mu$g/ml Säulenvolumen.

Bisherige Reinigungsverfahren wenden häufig die im Labor genutzten - relativ toxischen - Lösemittel (Acetonitril) und korrosiven, teuren Pufferkomponenten z.B. Tetraalkylammoniumsalze, Alkylsulfonate, Hexafluoraceton, Trifluoracetat an (E.P. Kroeff et al., J. Chrom., 461, (1989), 45-61). Diese Laufmittel führen bei stärker mit insulinähnlichen Substanzen verunreinigten Gemischen zu keiner befriedigenden präparativen Trennung im Hinblick auf Qualität, Ausbeute der Hauptkomponente und Gesamtwiederfindung (J. Rivier, R. McClintock; J. Chrom., 268 (1983) 112-119; Peter et al., J. Chrom. 408 (1987) 43-52).

Insuline aus vorherigen chemischen Umwandlungen wie beispielsweise aus stark sauren Esterspaltungen oder enzymatischen (Trans-)Peptidierungsprozessen, aus Kristallisationsreinigungen o.ä. enthalten zumeist Begleitstoffe mit ähnlichen Eigenschaften. Sie lassen sich durch die Wahl bestimmter pH-Werte über Ionenaustauschchromatographie reinigen, wenn genügend elektrische Ladungsunterschiede vorhanden sind (US 4 129 560). Der Nachteil dieser Methode liegt im Verdünnungseffekt und damit Verlust von Wertstoffen in den Überständen bei der Aufarbeitung der Fällungen, in der relativ langen Zykluszeit oder darin, daß die Gesamtwiederfindung und damit die Ausbeute geringer ist.

Präparative Trennungen lassen sich prinzipiell durch Vergrößern von Säuleninhalt, Beladungsmenge und Elutionsmitteldurchsatz erreichen. Die hier benötigten organischen Lösemittelmengen liegen z.B. bei Säulen mit Durchmessern >20 cm im m³-Maßstab. Zur analytischen HPLC verwendete Lösemittel (z.B. Acetonitril, DMF, Methanol, Dioxan, etc.) sind toxisch, so daß eine Anwendung dieser Methoden im präparativen Produktionsmaßstab aufwendige Schutzmaßnahmen erfordert.

Aufgabe der vorliegenden Erfindung war es ein Verfahren zur chromatographischen Reinigung von Insulinen und Insulinderivaten zu entwickeln, bei dem die biologische Aktivität der Insuline erhalten bleibt, hohe Reinheiten mit einem Chromatographieschritt erreicht werden, kurze Zykluszeiten, Säulenstandzeiten von mehr als 50 Chromatographiezyklen, Regenerierung der stationären Kieselgelphase ohne zeitraubende Packungsvorgänge und der Einsatz von untoxischen Lösemitteln erreicht wird, so daß ein präparativer Produktionsmaßstab möglich ist.

Es wurde nun ein Verfahren gefunden, durch das sich Insulin und Insulinderivate durch Chromatographie in wäßrigen, gepufferten Lösungsmitteln, die mit Wasser mischbare organische Lösungsmittel enthalten, an lipophil modifiziertem Kieselgel reinigen lassen, das dadurch gekennzeichnet ist, daß in den gepufferten Lösungsmitteln Zwitterionen gelöst sind oder der pH-Wert des Lösungsmittels in der Nähe des isoelektrischen Punkts, des zu reinigenden Insulins oder Insulinderivats liegt und Zwitterionen anwesend sind.

Überraschenderweise bewirken die Anwesenheit von Zwitterionen oder die Chromatographie bei einem pH-Wert des Lösungsmittels, der in der Nähe des isoelektrischen Punkts des zu reinigenden Insulins oder Insulinderivats liegt und die Anwesenheit von Zwitterionen, nicht nur eine gute Trennung von Wertprodukt und Verunreinigungen, sondern auch eine gute Ablösung der Proteine von der stationäre Phase (lipophil modifiziertes Kieselgel). Die erreichten Trennungen erlauben es, selbst stark mit insulinähnlichen Komponenten verunreinigte Insulinlösungen zu reinigen, wie z.B. die Trennung von A21-Desamidoinsulin von Insulin und Insulinderivaten. Ferner werden durch die günstige Ablösung der Proteine von der Festphase lange Standzeiten der Säulenpackungen erreicht und eine hohe Gesamtwiederfindungsrate der Insuline ermöglicht.

Die Erfindung betrifft somit ein Verfahren zur Reinigung von Insulin und/oder Insulinderivaten durch Chromatographie in wäßrigen, gepufferten Lösungsmitteln, die mit Wasser mischbare organische Lösungsmittel enthalten, an lipophil modifiziertem Kieselgel, das dadurch gekennzeichnet ist, daß in den gepufferten Lösungsmitteln Zwitterionen gelöst sind oder der pH-Wert des Lösungsmittelgemisches in der Nähe des isoelektrischen Punkts des zu reinigenden Insulins oder Insulinderivats liegt und Zwitterionen anwesend sind.

In dem erfindungsgemäßen Verfahren können alle Insuline eingesetzt werden, wie z.B. Insuline aller Species, die tierischen oder menschlichen Ursprungs sind, Insulinvorläufer, wie z.B. Proinsuline oder Präproinsuline, oder rekombinante Insuline oder Insulinderivate, die von genetisch modifizierten Mikroorganismen exprimiert werden. Ferner können auch Insulinderivate eingesetzt werden, die beispielsweise durch chemische oder enzymatische Derivatisierung hergestellt wurden, z.B. Des-Phe-B1-Insulin, Insulin-β-Ketensulfonat, Diargininsulin(B31, B32), Monoargininsulin oder Diphenylalaninsulin(B31, B32) (US 4 601 852).

Bevorzugt werden Insulinderivate der Formel I

```
              S————S              A21
       ┌────────────────────────────────┐
  H————│      A-KETTE           Asn      │————OH
       └────────────────────────────────┘
              S        S
              │        │                          ( I )
              S        S
       B2     │        │             B29
       ┌────────────────────────────────┐
  Y————│  Val   B-KETTE           Lys   │————R³⁰-Xₙ
       └────────────────────────────────┘
```

in welcher

$R^{30}$     für den Rest einer genetisch kodierbaren L-Aminosäure,

X     für eine Hydroxygruppe, eine physiologisch unbedenkliche organische Gruppe basischen Charakters mit bis zu 50 C-Atomen, eine genetisch kodierbare L-Aminosäure und deren gegebenenfalls vorhandene endständige Carboxylfunktion frei, als Esterfunktion, als Amidfunktion, als Lacton oder zu $CH_2OH$ reduziert vorliegen kann,

n     für eine ganze Zahl von 0 bis 10,

Y     für Wasserstoff oder L-Phenylalanin steht

und die A- und B-Ketten die Sequenzen tierischen oder menschlichen Insulins sind, eingesetzt.

Besonders bevorzugt werden Insulinderivate der Formel I, in welcher

$R^{30}$     für L-Alanin oder L-Threonin und

X     für eine oder mehrere L-Aminosäuren aus der Gruppe

L-Arginin, L-Lysin oder L-Phenylalanin steht, eingesetzt.

Die Insuline und Insulinderivate können sowohl in verhältnismäßig unreinem Zustand, als auch in vorgereinigter Form (z.B. durch Gelchromatographie), eingesetzt werden. Insulin ist nach mehrfacher Kristallisation und auch nach Gelchromatographie noch mit Insulin-ähnlichen Begleitstoffen sehr ähnlichen Molekulargewichtes verunreinigt, die sich bei passend gewähltem pH in ihrem Ladungszustand untereinander und vom Insulin unterscheiden, aber mit Insulin Komplexe bilden (US 4 129 560). Beispiele solcher Substanzen sind: Desamidoinsuline, Arginin- und Diargininsulin, Insulin-ethylester und andere.

Unter lipophil modifiziertem Kieselgel wird ein Kieselgel verstanden, auf das eine hydrophobe Matrix aufgebracht wurde. Beispiele für eine hydrophobe Matrix sind Alkane mit einer Kettenlänge von 3 bis 20 Kohlenstoffatomen. Besonders bevorzugte lipophil modifizierte Kieselgelmaterialien sind beispielsweise:

- (R)Nucleosil, Firma Macherey & Nagel: sphärische und nicht sphärische Materialien verschiedener Korngrößen bis zu 45 μm, 100 Å Porenweite, C8- bzw. C18-modifiziert.
- (R)Lichroprep, Firma Merck: nicht sphärische und sphärische Materialien verschiedener Korngrößen bis 40 μm, 60 - 250 Å Porenweite, C8-C18-modifiziert;
- (R)Lichrospher Select B, Firma Merck: sphärisches Material bis 25 μm Korngröße, C8-modifiziert;
- (R)Waters Prep, C18-modifiziert, 50 - 105 μm nichtsphärisch, 100 Å Porenweite;
- (R)Zorbax Pro10, Firma DuPont: C8-modifiziert, 10 μm, sphärisch, 100 Å Porenweite;
- (R)Kromasil, Firma EKA Nobel: C4-, C8-C18-modifiziert, bis 16 μm, sphärisch, 100, 150 oder 200 Å Porenweite.

Zwitterionen sind Verbindungen, die Protonen aufnehmen und auch abspalten können, d.h. in saurer Lösung Kationen und in alkalischer Lösung Anionen bilden, wie beispielsweise α-Aminosäuren, Betain oder Betainderivate. Bevorzugte Zwitterionen sind Glycin, Glutamin oder Betain (N-Trimethyl-glycin). Besonders bevorzugt ist Glycin.

Der isoelektrische Punkt (IEP) eines Insulins oder Insulinderivates ist derjenige pH-Wert einer Lösung des Insulins, in der die Anzahl der kationischen Ladungen und anionischen Ladungen des gelösten Insulins gleich Null ist. Beispielsweise liegt der IEP von Schweineinsulin zwischen pH 5,3 und 5,4 (H. Neurath, K. Bailey, Protein Hormones, Vol. II/A, The Proteins, S. 636). Mit dem Begriff "in der Nähe des isoelektrischen Punkts" sind insbesondere pH-Werte gemeint, die ca. um 1 pH-Einheit über oder unter dem IEP des zu reinigenden Insulins liegen. Besonders bevorzugt sind pH-Werte, die bis zu 0,5 pH-Einheiten über oder unter dem IEP liegen.

Die Elutionsmittel enthalten eine Puffersubstanz, um den pH-Wert des Elutionsmittels konstant zu halten. Geeignete Puffersubstanzen sind in der Literatur bekannt, beispielsweise Phosphate, Alkali- oder Erdalkalimetallsalze, wie Natriumcitrat oder Kaliumacetat, Ammoniumcitrat, -acetat, -sulfat oder -chlorid. Ferner enthalten die Elutionsmittel mit Wasser mischbare organische Lösungsmittel wie beispielsweise Alkohole, Ketone, Methylacetat, Dioxan, Dimethylsulfoxid oder Acetonitril. Bevorzugt werden Alkohole wie n- oder iso-Propanol, Methanol, Ethanol oder Methylacetat.

Die Konzentration der mit Wasser mischbaren organischen Lösungsmittel liegt zwischen 1 und 90 %, bevorzugt zwischen 10 und 60 %, besonders bevorzugt zwischen 10 und 35 %. Die Konzentration der Puffersubstanz liegt zwischen 1 mMol/l und 2 Mol/l, bezogen auf Wasser als Lösungsmittel, bevorzugt zwischen 25 mMol/l und 1 Mol/l. Die Konzentration der Zwitterionen kann in einem weiten Bereich schwanken. Vorteilhafte Mengen liegen zwischen 10 mMol/l und 1 Mol/l, bezogen auf Wasser als Lösungsmittel, bevorzugt zwischen 20 mMol/l und 500 mMol/l.

Die Temperatur während der Chromatographie liegt zwischen 0°C und 50°C, vorzugsweise zwischen 15 und 30°C, besonders bevorzugt zwischen 15 und 20°C. Der Betriebsdruck während der Chromatographie ist weitgehend konstant. Die Chromatographie kann mit unterschiedlichem Druck durchgeführt werden, z.B. kann die Chromatographie bei einem Druck von 5 bis 400 bar durchgeführt werden, insbesondere bei 20 bis 100 bar.

Die Beladung der Säulen, Chromatographie und Elution der Insuline und Insulinderivate erfolgt nach bekannten, üblichen, technischen Methoden. Die Beladung der Säule mit der zu reinigenden Insulinlösung erfolgt bevorzugt mit wäßrig - alkoholischer oder rein wäßriger Pufferlösung. Die Insulinlösung hat einen Proteinanteil der zwischen 1 und 10 % liegt, bevorzugt bei 3 %.

Die Elution der Insuline erfolgt mit dem erfindungsgemäßen Verfahren bei konstanter Konzentration der Puffersubstanzen (isokratisch) oder durch Veränderung des mit Wasser mischbaren organischen Lösungsmittelanteils am Puffer. Die Veränderung des organischen Lösungsmittelanteils erfolgt in der Weise, daß die Konzentration des verwendeten organischen Lösungsmittels in Abhängigkeit vom Elutionsvolumen steigt, und zwar vorzugsweise in linearer Abhängikeit.

Die Abtrennung des Insulins nach der Chromatographie aus den Eluaten geschieht durch Ausfällung mit Zink oder durch Kristallisation. Dabei kann die Lösung wahlweise vorher mittels Vakuumdestillation weitgehend vom Lösungsmittel befreit oder dessen Konzentration durch Verdünnen mit Wasser reduziert werden. Auf jeden Fall sollte die Lösungsmittelkonzentration vor der Fällung oder Kristallisation bei 10 % oder darunter liegen, um den Proteingehalt im Überstand auf <50 mg/l zu halten. Die entstandenen reinen Insulinniederschläge lassen sich durch Dekantieren, Zentrifugieren oder Filtration isolieren und trocknen.

Das erfindungsgemäße Verfahren eignet sich nicht nur zur analytischen Chromatographie, sondern auch zur präparativen Chromatographie, insbesondere wenn das erfindungsgemäße Verfahren mit einer präparativen HPLC-Anlage durchgeführt wird.

Unter dem Begriff "präparative Chromatographie" wird ein Reinigungsverfahren verstanden, mit dem Ziel Reinprodukte zu gewinnen und nicht nur zu analysieren. Die Menge der Reinprodukte kann in weiten Grenzen schwanken, beispielsweise von 1 mg bis 50 kg, bevorzugt zwischen 50 mg und 15 kg.

In den folgenden Beispielen wird das erfindungsgemäße Verfahren im einzelnen beschrieben. Prozentangaben beziehen sich auf das Gewicht, sofern nicht anders angegeben.


**Beispiel 1**

| | |
|---|---|
| Puffer A: | 0,2 M Ammoniumsulfat, 0,1 M Glycin, 0,05 M Natriumcitrat, pH 5,5, rein wäßrig; |
| Puffer B: | 0,1 M Ammoniumsulfat, 0,1 M Glycin, 0,05 M Natriumcitrat, pH 5,5, Wasser/n-Propanol im Verhältnis 1:1. |
| Sorbens: | Nucleosil C18, 15 - 25 $\mu$m sphärisch, 100 Å Porenweite, Fa. Machery & Nagel, Düren; |
| Säulenabmessung: | 40 mm x 250 mm. |

Die Beladung der Säule erfolgt mit 3,5 g Humaninsulin (HI) - gewonnen aus Trifluoressigsäurespaltung von Humaninsulin-B30-di-tert.butyl-ester/ether - mit einem Proteinanteil von 79,1 %. Die Elution des Insulins erfolgt dadurch, daß die Pufferlösung A und B mit einer geeigneten Hochdruckpumpe mit Mischeinrichtung gemischt werden, so daß ein Propanolgradient von 14 bis 20 % erzeugt wird. Die Elution des Insulins erfolgt bei etwa 17,5 % Propanol nach 23 Minuten, wenn mit 46 ml/min gepumpt wird.

Nach Fraktionierung und Isolierung von kristallisiertem Humaninsulin (HI) erhält man ein Produkt mit 97 % Protein in 88,5 %iger Ausbeute in der Hauptfraktion und 7,5 % Ausbeute an Nebenfraktion mit 85,7 % Proteinanteil. Die Gesamtwiederfindung beträgt somit 96,0 % des vorgelegten Insulins.

**Beispiel 2**

| Puffer A: | 0,1 M Ammoniumsulfat, 0,1 M Glycin, 0,025 M Natriumcitrat, pH 5,5, rein wäßrig; |
|---|---|
| Puffer B: | 0,05 M Ammoniumsulfat, 0,1 M Glycin, 0,025 M Natriumcitrat, pH 5,5, Wasser/n-Propanol im Verhältnis 1:1. |
| Sorbens: | Nucleosil C18-P, 15 - 25 $\mu$m sphärisch, 100 Å Porenweite, Fa. Macherey & Nagel; |
| Säulenabmessung: | 40 mm x 250 mm. |

Die Säule wird mit 7,0 g Humaninsulin (HI) (86,1 % Proteinanteil) aus einer Esterspaltung von Humaninsulin-di-tert.butyl-ester/ether in Form einer 3 %igen Lösung in 0,1 M Glycin/HCl-Puffer, pH 2,8, mit Hilfe einer Hochdruckpumpe beladen. Anschließend erfolgt die Elution mit den oben beschriebenen Pufferlösungen unter Druck im n-Propanolgradient. Innerhalb von 60 Minuten steigt die Propanolkonzentration von 14 auf 17,5 % an. Nach Fraktionierung und Kristallisation in der o.g. Weise erhält man ein Produkt mit 98,7 % Proteinanteil. Die Ausbeute an gereinigtem Humaninsulin liegt bei 91 % des eingesetzten Insulins.

**Beispiel 3**

| Puffer A: | 0,1 M Ammoniumsulfat, 0,1 M Glycin, 0,025 M Natriumacetat, pH 5,5, rein wäßrig; |
|---|---|
| Puffer B: | 0,05 M Ammoniumsulfat, 0,1 M Glycin, 0,025 M Natriumacetat, pH 5,5, Wasser/n-Propanol im Verhältnis 1:1. |
| Sorbens: | Lichrospher Select B, C8, 15 - 25 $\mu$m, 60 Å Porenweite, Fa. Merck. |
| Säulenabmessung: | 50 mm x 250 mm. |

Die Säule wird mit einer Lösung von 10 g Reaktionsgemisch aus der Umamidierung von Schweineinsulin mit Trypsin, gelöst in 200 ml 0,1 M Glycin/HCl-Puffer, pH 2,8, beladen. Innerhalb von 120 Minuten werden bei einem Fluß von 40 ml/min. und Erhöhung der Propanolkonzentration von 14 auf 30 % die einzelnen Proteinkomponenten getrennt eluiert. Man erhält nach Kristallisation bzw. Fällung und Trocknung als Hauptfraktion Humaninsulin-B30-di-tert.butylthreoninester/ether mit einer Reinheit von >97 % mit einer Ausbeute von 93 %, bezogen auf das eingesetzte Insulin.

**Beispiel 4**

| Puffer A: | 0,1 M Natriumchlorid, 0,1 M Glycin, 0,025 M Natriumacetat, pH 5,5, rein wäßrig; |
|---|---|
| Puffer B: | 0,05 M Natriumchlorid, 0,1 M Glycin, 0,025 M Natriumacetat, pH 5,5, Wasser/n-Propanol im Verhältnis 1:1. |
| Sorbens: | Zorbax Pro10, C8, 10 $\mu$m, Fa. DuPont. |
| Säulenabmessung: | 5 cm x 25 cm. |

Es wurden 7,5 g HI aus Trifluoressigsäurespaltung von Humaninsulin-B30-di-tert.butylthreoninester/ether in 100 ml 0,1 M Glycin/HCl-Lösung auf die Säule gepumpt und bei einem Fluß von 80 ml/Min. eluiert. Die Konzentration von Puffer B stieg innerhalb von 60 Minuten von 18 auf 25 % - Die Retentionszeit (Rt) betrug hier ca. 37 Minuten. Aus der Hauptfraktion ließen sich nach Kristallisation und Trocknung 96,8 % HI vom Einsatz mit >97 % Reinheit isolieren, die Nebenfraktion enthielt 2,2 % HI mit <50 % Reinheit.

**Beispiel 5**

| Puffer A: | 0,2 M Natriumsulfat, 0,1 M Glycin, 0,03 M Ammoniumacetat, pH 5,5, 10 % Methylacetat; |
|---|---|
| Puffer B: | 0,05 M Natriumsulfat, 0,1 M Glycin, 0,03 M Ammoniumacetat, pH 5,5, 20 % Methylacetat. |
| Sorbens: | Kromasil C8, 13 $\mu$m, 100 Å Porenweite, Fa. EKA Nobel. |

Säulenabmessung:     5 cm x 25 cm.

Es wurden 8 g Rinderinsulin pro Liter Säulenvolumen mit Hilfe von 0,1 M Glycin/HCl-Puffer auf die Säule - äquilibriert bei 30 % Puffer B - gebracht. Innerhalb von 90 Minuten stieg die B-Pufferkonzentration auf 80 % (= 18 % Methylacetat); Rinderinsulin eluierte nach 65 Minuten mit einer Reinheit von >97,5 % in der Hauptfraktion (63,5 % Ausbeute) und wurde nach Verdünnen mit Wasser mit Zinkchlorid gefällt. Die Gesamtwiederfindung betrugt 92,5 %.

**Beispiel 6**

Puffer A:          0,1 M Ammoniumsulfat, 0,1 M Glycin, 0,025 M Natriumacetat, pH 5,5, 5 % n-Propanol;

Puffer B:          0,05 M Ammoniumsulfat, 0,1 M Glycin, 0,025 M Natriumacetat, pH 5,5 Wasser/n-Propanol im Verhältnis 1:1.

Sorbens:          Kromasil C8, 13 $\mu$m, 100 Å Porenweite, Fa. EKA Nobel.

Säulenabmessung:     5 cm x 25 cm.

Die Beladung der Säule betrug 4 g (= 8 g/l) Humaninsulin aus Trifluoressigsäurespaltung von Humaninsulin-B30-di-tert.butylthreoninester/ether mit einer Reinheit von ca. 92 %. In einem Gradient von 18 auf 25 % Puffer B innerhalb von 90 Minuten eluierte das Humaninsulin nach 45 Minuten mit einer Reinheit von >97 % in der Hauptfraktion (Ausbeute 91,8 %) und 80,5 % in der Nebenfraktion (Ausbeute 4,7 %).

**Beispiel 7**

Puffer A:          0,1 M Ammoniumsulfat, 0,1 M Glycin, 0,025 M Natriumacetat, pH 5,5, 10 % n-Propanol;

Puffer B:          0,05 M Ammoniumsulfat, 0,1 M Glycin, 0,025 M Natriumacetat, pH 5,5, Wasser/n-Propanol im Verhältnis 1:1.

Sorbens:          Kromasil C8, 13 $\mu$m, 100 Å Porenweite, Fa. EKA Nobel.

Säulenabmessung:     10 cm x 40 cm.

Auf die Säule wurden 18 g Schweineinsulin in 1,8 l 0,1 M Glycin/HCl-Puffer, pH 3,0, mit 5 % n-Propanol gegeben. Der Gradient verlief von 9 % Puffer B (= 13,6 % n-Propanol) auf 11 % Puffer B (= 14,4 % n-Propanol) in 70 Minuten. Schweineinsulin eluierte nach 50 Minuten mit einem Gehalt an Protein >98 % in der Hauptfraktion (89 % Ausbeute). Die Gesamtwiederfindung betrug 96,8 % vom Einsatz.

**Beispiel 8**

Puffer A:          0,2 M Ammoniumchlorid, 0,1 M Glycin, 0,025 M Natriumcitrat, 5 % n-Propanol, pH 5,5;

Puffer B:          wie Puffer A, jedoch Zusatz von 50 % n-Propanol.

Sorbens:          Kromasil C8, 13 $\mu$m, 100 Å Porenweite, Fa. EKA Nobel, Schweden.

Säulenabmessung:     50 mm x 250 mm.

4 g gentechnisch hergestelltes Humaninsulin (89,5 % Proteinanteil) werden in 0,1 M Glycin-Puffer, pH 3,0, als 2 %ige Proteinlösung gelöst und durch eine Hochdruckpumpe auf die Säule gepumpt. Die Elution erfolgt durch einen linearen Gradienten von 13 % auf 16 % n-Propanol innerhalb 70 Minuten bei einem Fluß von 80 ml/min. Nach Fraktionierung und Kristallisation erhält man in der Hauptfraktion ein Humaninsulin mit einem Proteingehalt von 98 % mit einer Ausbeute von 93 %. Die Gesamtwiederfindung liegt bei 98 % bezogen auf den Einsatz.

**Beispiel 9**

Puffer A:          0,2 M Ammoniumsulfat, 0,1 M Glycin, 0,025 M Ammoniumcitrat, 5 % Ethanol, pH 5,5;

Puffer B:          wie Puffer A, jedoch Zusatz von 50 % Ethanol.

Sorbens:          Kromasil C8, 10 $\mu$m, 100 Å Porenweite, Fa. EKA Nobel, Schweden.

Säulenabmessung:     50 mm x 250 mm.

In 250 ml Glycin-Puffer, pH 3,0 werden 4,5 g gentechnisch hergestelltes Humaninsulin (86,2 % Proteinanteil) gelöst und dann auf die Säule gepumpt. Zur Chromatographie des Proteins werden durch zwei Hochdruckpumpen ein linearer Gradient aus den beiden Puffern A und B erzeugt. Die Elution erfolgt nach 100 Minuten bei einer Ethanolkonzentration von 33 % und einem konstanten Fluß von 80 ml/min. Nach Fraktionierung und Kristallisation erhält man in der Hauptfraktion ein Humaninsulin mit einem Proteingehalt von 96 % bei einer Gesamtwiederfindungsrate von 93 % bezogen auf das Einsatzprodukt.

**Beispiel 10**

Puffer A: 0,2 M Ammoniumchlorid, 0,1 M Glycin, 0,025 M Natriumcitrat, 10 % Methylacetat, pH 5,5;
Puffer B: gleiche Salzkonzentration wie in Puffer A, jedoch 20 % Methylacetat.
Sorbens: Kromasil C8, 10 $\mu$m, 100 Å Porenweite, Fa. EKA Nobel, Schweden.

Für die Chromatographie werden 4,6 g rohes Humaninsulin, das aus der Aufarbeitung von gentechnisch hergestelltem Insulin stammt, in 200 ml wäßrigem Glycin-Puffer aufgelöst und dann auf eine 50 x 250 mm HPLC-Säule gepumpt. Das Säulenmaterial wird zuvor durch Zusammenmischen der Puffer A und B mittels zwei Hochdruckpumpen auf eine Methylacetatkonzentration von 13 % Methylacetat äquilibriert. Nach der Aufgabe erfolgt die Elution des Humaninsulins durch einen linearen Gradienten von 13 % auf 17 % Methylacetat innerhalb 90 Minuten. Die Isolierung des Hauptproduktes erfolgt durch Kristallisation aus der Elutionslösung. Dabei erhält man 3,8 g Humaninsulin mit einem Proteinanteil von 96 %. Die Gesamtwiederfindungsrate, bezogen auf das Ausgangsmaterial, beträgt dabei 90 %.

**Beispiel 11**

Puffer A: 0,2 M Ammoniumsulfat, 0,1 M Betain, 0,05 M Zitronensäure, pH 5,5 mit Natronlauge (rein wäßrig);
Puffer B: 0,1 M Ammoniumsulfat, 0,1 M Betain, 0,05 M Zitronensäure, pH 5,5 mit Natronlauge (Wasser/iso-Propanol Verh. 1:1);
Sorbens: Nucleosil C18, 15 - 25 $\mu$m sphärisch, 100 Å;
Säule: 40 mm x 250 mm.
Fluß: 45 ml/Min.

Die Säule wurde mit 3 g (79 % Proteinanteil) Humaninsulin aus einer Esterspaltung von Humaninsulin-di-tert.butyl-ester/ether in Form einer 3 %igen Lösung in 0,1 M Betain/HCl-Puffer, pH 3,0, mit Hilfe einer Hochdruckpumpe beladen. Anschließend wurde mit dem o.g. Puffersystem bei 44 % Puffer B isokratisch eluiert. Die Retentionszeit betrug ca. 35 Minuten. Nach Fraktionierung wurde in der Hauptfraktion 98,1 % Humaninsulin mit 80,5 % Ausbeute erhalten. Die Gesamtwiederfindung betrug 90,5 %.

**Beispiel 12**

Puffer A: 0,1 M Ammoniumsulfat, 0,1 M Glutaminsäure, 0,05 M Zitronensäure, pH 5, 5 mit Natronlauge (rein wäßrig);
Puffer B: 0,1 M Ammoniumsulfat, 0,1 M Glutaminsäure, 0,05 M Zitronensäure, pH 5,5 mit Natronlauge (Wasser/n-Propanol: 1:1);
Sorbens: Nucleosil C18-P, 15 - 25 $\mu$m sphärisch, 100 Å;
Säule: 50 mm x 250 mm.
Fluß: 60 ml/Min.

Die Säule wurde mit 6 g (73 % Proteinanteil) Humaninsulin aus einer Esterspaltung von Humaninsulin-di-tert.butyl-ester/ether in Form einer 3 %igen Lösung in 0,1 M Essigsäure, pH 3,0, mit Hilfe einer Hochdruckpumpe beladen. Anschließend wurde mit dem o.g. Puffersystem bei 25 % - 28 % Puffer B im Gradient eluiert. Die Retentionszeit betrug ca. 25 Minuten. Nach Fraktionierung wurde in der Hauptfraktion 98,6 % Humaninsulin mit 65 % Ausbeute erhalten. Die Gesamtwiederfindung betrug 88,5 %.

**Beispiel 13**

Puffer A: 0,15 M Ammoniumsulfat, 0,10 M Triethylamin, pH 6,5 mit Schwefelsäure (rein wäßrig);
Puffer B: 0,08 M Ammoniumsulfat, 0,05 M Triethylamin, pH 6,5 mit Schwefelsäure (Wasser/2-Propanol: 1:1);
Sorbens: Nucleosil C18-P, 15 - 25 $\mu$m sphärisch, 100 Å;
Säule: 50 mm x 250 mm.
Fluß: 60 ml/Min.

Nach Beladung der Säule mit 5 g Humaninsulin aus Trifluoressigsäurespaltung von Humaninsulin-di-tert.butylthreoninester/ether in einer 3 %igen Lösung wurde zwischen 32 und 42 % Puffer B eluiert. Die Retentionszeit betrug 40 Minuten. Nach Fraktionierung und Kristallisation wurden 72 % in der Hauptfraktion mit einer Reinheit von 92,7 % erhalten. Die Gesamtwiederfindung lag bei 76 % der eingesetzten Menge.

**Beispiel 14**

Puffer A:     0,1 M Zitronensäure, 0,2 M Ammoniumsulfat, pH 2,5 mit Salzsäure, rein wäßrig
Puffer B:     0,1 M Zitronensäure, 0,1 M Ammoniumsulfat, pH 2,5 mit Salzsäure, Wasser/n-Propanol: 1:1.
Sorbens:     Nucleosil C18, Fa. Macherey & Nagel
Säule:        40 x 250 mm
Fluß:         45 ml/Min.

Nach Beladung der Säule mit 3 g Humaninsulin aus Trifluoressigsäurespaltung von Humaninsulin-di-tert.butylthreoninester/ether in Form einer 3 %igen Lösung in 0,1 M Glycinpuffer pH 3,0 wurde zwischen 34 und 35 % Puffer B eluiert. Die Retentionszeit betrug 45 Minuten. Nach Fraktionierung, Kristallisation und Trocknung wurden 57 % der Einsatzmenge in der Hauptfraktion mit einer Reinheit von 94,8 % und 16 % in der Nebenfraktion (Reinheit 84 %) isoliert.

**Beispiel 15:**

Puffer A:     0,1 M Zitronensäure, 0,2 M Ammoniumsulfat, pH 3,5 mit Salzsäure, rein wäßrig
Puffer B:     0,1 M Zitronensäure, 0,1 M Ammoniumsulfat, pH 3,5 mit Salzsäure, Wasser/n-Propanol: 1:1.
Sorbens:     Nucleosil C18, Fa. Macherey & Nagel
Säule:        40 x 250 mm
Fluß:         45 ml/Min.

Säulenbeladung erfolgt wie in Beispiel 14. Die Retentionszeit betrug ca. 40 Minuten. Nach Fraktionierung, Kristallisation und Trocknung wurden 51 % eingesetztes Humaninsulin mit einer Reinheit von 97,5 % in der Hauptfraktion und 4 % in der Nebenfraktion (68 % Reinheit) gefunden.

**Beispiel 16**

Puffer A:     0,1 M Tris, 0,1 M Glycin, 0,1 M Ammoniumchlorid, pH 8,5 mit Salzsäure, rein wäßrig;
Puffer B:     0,1 M Tris, 0,1 M Glycin, 0,1 M Ammoniumchlorid, pH 8,5 mit Salzsäure, Wasser/n-Propanol: 1:1.
Sorbens:     Kromasil C8, 13 $\mu$m, 100 Å
Säule:        50 mm x 250 mm
Fluß:         60 ml/Min.

Die auf 33 % Puffer B equilibrierte Säule wurde mit 6 g Humaninsulin aus Trifluoressigsäurespaltung (s. Beispiel 14) in Form einer 3 %igen Lösung in 0,1 M Trispuffer, pH 8,5, beladen. Elution erfolgte nach ca. 35 Minuten. Nach Fraktionierung und Kristallisation enthielt die Hauptfraktion 96,6 %iges Humaninsulin mit einer Ausbeute von 86 %. Die Gesamtwiederfindung lag bei 95 %.

**Beispiel 17:**

Puffer A:     0,1 M Ammoniumchlorid, 0,025 M Zitronensäure, pH 5,5 mit Ammoniak, 5 Vol.-% n-Propanol
Puffer B:     0,1 M Ammoniumchlorid, 0,025 M Zitronensäure pH 5,5 mit Ammoniak, 50 Vol.-% n-Propanol
Sorbens:     Kromasil C8, 13 $\mu$m, 100 Å
Säule:        50 mm x 250 mm
Fluß:         60 ml/Min.

Die Säule wurde wie schon beschrieben mit 6 g Humaninsulin aus Trifluoressigsäurespaltung (s. Beispiel 14) beladen. Innerhalb eines Gradienten von 21 % auf 25 % Puffer B in 60 Minuten wurde Insulin mit einer Retentionszeit von 40 Minuten eluiert. Die Hauptfraktion enthielt 91 % des Einsatzproduktes mit einer Reinheit von 97,5 %. Die Gesamtwiederfindung lag bei 96 %.

**Beispiel 18:**

Puffer A:     0,1 M Kaliumchlorid, 0,025 M Zitronensäure, pH 5,5 mit Kalilauge, 5 Vol.-% n-Propanol
Puffer B:     0,1 M Kaliumchlorid, 0,025 M Zitronensäure, pH 5,5 mit Kalilauge, 50 Vol.-% n-Propanol
Sorbens:     Kromasil C8, 13 $\mu$m, 100 Å

Säule:      50 mm x 250 mm

Fluß:      60 ml/Min.

Beladung und Gradient wie in Beispiel 17. Elution erfolgte nach 35 Minuten. Die Hauptfraktion enthielt 90 % der Einsatzmenge an Humaninsulin mit einer Reinheit von 96,5 %. Die Gesamtwiederfindung lag bei 93 %.

**Beispiele 19 bis 24:**

In den Beispielen 19 bis 24 wurde die Chromatographie unter den gleichen Bedingungen wie in Beispiel 14 durchgeführt. Nur die Puffer bzw. die Zwitterionmenge wurde verändert.

**Beispiel 19:**

Puffer A:     0,15 M Ammoniumsulfat, 0,1 M Triethylamin, pH 7,0 mit Schwefelsäure;

Puffer B:     0,08 M Ammoniumsulfat, 0,05 M Triethylamin, pH 7,0 mit Schwefelsäure.

Ergebnis:    95,2 % Reinheit der Hauptfraktion; 67 % Ausbeute in der Hauptfraktion.

**Beispiel 20:**

Puffer wie in Beispiel 16 jedoch ohne 0,1 M Glycin

Ergebnis:    96,1 % Reinheit in der Hauptfraktion; 65 % Ausbeute in der Hauptfraktion und 25 % in der Nebenfraktion.

**Beispiel 21:**

Puffer wie in Beispiel 15 mit zusätzlich 0,1 M Glycin.

Ergebnis:    96 % Reinheit in der Hauptfraktion; 66 % Ausbeute in der Hauptfraktion und 6 % in der Nebenfraktion.

**Beispiel 22:**

Puffer A:     0,1 M Ammoniumchlorid, 0,025 M Zitronensäure, pH 4,5 mit Ammoniak, 5 Vol.-% n-Propanol, 0,1 M Glycinbetain

Puffer B:     wie Puffer A aber zusätzlich 50 % n-Propanol

Ergebnis:    98,1 % Reinheit in der Hauptfraktion; 88 % Ausbeute in der Hauptfraktion und 6 % in der Nebenfraktion.

**Beispiel 23:**

Puffer wie in Beispiel 22, aber pH 5,4 und 0,1 M Glycin statt Glycinbetain.

Ergebnis:    97,9 % Reinheit in der Hauptfraktion; 92 % Ausbeute in der Hauptfraktion und 8 % in der Nebenfraktion.

**Beispiel 24:**

Puffer wie in Beispiel 13, aber pH 6,0 und zusätzlich 0,1 M Glycin.

Ergebnis:    94,3 % Reinheit in der Hauptfraktion; 77 % Ausbeute in der Hauptfraktion und 13 % in der Nebenfraktion.

**Beispiel 25:**

Die Tabelle 1 gibt eine Übersicht über Ausbeute und Reinheit von Insulinen in Abhängigkeit von pH-Wert und/oder Zwitterion im Elutionsmittel.

## Tabelle 1

| pH-Wert | Zwitterion | Reinheit Hauptfraktion | Ausbeute Hauptfr. | Nebenfr. | Bsp. Nr. |
|---|---|---|---|---|---|
| 2,5 | - | 94,8 | 57 | 16 | 14 |
| 3,5 | - | 97,5 | 51 | 4 | 15 |
| 6,5 | - | 92,7 | 72 | 4 | 13 |
| 7,0 | - | 95,2 | 67 | - | 19 |
| 8,5 | - | 96,1 | 65 | 25 | 20 |
| 5,5 | - | 97,5 | 91 | 5 | 17 |
| 5,5 | - | 96,5 | 90 | 3 | 18 |
| 3,5 | Gly | 96,0 | 66 | 6 | 21 |
| 4,5 | Glycinbetain | 98,1 | 88 | 6 | 22 |
| 5,4 | Gly | 97,9 | 92 | 8 | 23 |
| 5,5 | Gly | 98,0 | 93 | 5 | 8 |
| 6,0 | Gly | 94,3 | 77 | 13 | 24 |
| 8,5 | Gly | 96,6 | 86 | 9 | 16 |

**Patentansprüche**

1. Verfahren zur Reinigung von Insulin und/oder Insulinderivaten durch Chromatographie in wäßrigen, gepufferten Lösungsmitteln, die mit Wasser mischbare organische Lösungsmittel enthalten, an lipophil modifiziertem Kieselgel, dadurch gekennzeichnet, daß in den gepufferten Lösungsmitteln Zwitterionen gelöst sind oder der pH-Wert des Lösungsmittelgemisches in der Nähe des isoelektrischen Punktes des zu reinigenden Insulins oder Insulinderivats liegt und Zwitterionen anwesend sind.

2. Verfahren nach Anspruch 1, dadurch gekennzeichnet, daß der pH-Wert des Lösungsmittelgemisches bis zu 1 pH-Einheit unter oder über dem isoelektrischen Punkt des zu reinigenden Insulins oder Insulinderivats liegt.

3. Verfahren nach Anspruch 2, dadurch gekennzeichnet, daß der pH-Wert des Lösungsmittelgemisches bis zu 0,5 pH-Einheiten unter oder über dem isoelektrischen Punkt liegt.

4. Verfahren nach einem oder mehreren der Ansprüche 1 bis 3, dadurch gekennzeichnet, daß als Zwitterionen $\alpha$-Aminosäuren oder Betaine eingesetzt werden.

5. Verfahren nach Anspruch 4, dadurch gekennzeichnet, daß als Zwitterion Glycin, Glutaminsäure oder Glycinbetain eingesetzt werden.

6. Verfahren nach einem oder mehreren der Ansprüche 1 bis 5, dadurch gekennzeichnet, daß Insulinderivate der Formel I

```
            S———S              A21
    ┌─────────────────────────────┐
H───┤    A-KETTE          Asn    ├──OH
    └─────────────────────────────┘
         S      S
         |      |                        (I)
         S      S
    B2   |      |              B29
    ┌─────────────────────────────┐
Y───┤ Val  B-KETTE         Lys  ├──R³⁰-Xₙ
    └─────────────────────────────┘
```

in welcher

R$^{30}$      für den Rest einer genetisch kodierbaren L-Aminosäure,

X      für eine Hydroxygruppe, eine physiologisch unbedenkliche organische Gruppe basischen Charakters mit bis zu 50 C-Atomen, eine genetisch kodierbare L-Aminosäure und deren gegebenenfalls vorhandene endständige Carboxylfunktion frei, als Esterfunktion, als Amidfunktion, als Lacton oder zu $CH_2OH$ reduziert vorliegen kann,

n      für eine ganze Zahl von 0 bis 10

Y      für Wasserstoff oder L-Phenylalanin steht und die A- und B-Ketten die Squenzen tierischen oder menschlichen Insulins sind,

verwendet werden.

7. Verfahren nach Anspruch 6, dadurch gekennzeichnet, daß in Formel I

R$^{30}$      für L-Alanin oder L-Threonin und

X      für eine oder mehrere L-Aminosäuren aus der Gruppe

L-Arginin, L-Lysin oder L-Phenylalanin steht.

8. Verfahren nach einem oder mehreren der Ansprüche 1 bis 7, dadurch gekennzeichnet, daß eine präparative Hochdruckflüssigkeitschromatographie-Anlage eingesetzt wird.

**Claims**

1. A process for the purification of insulin and/or insulin derivatives by chromatography in aqueous, buffered solvents which contain water-miscible organic solvents, on lipophilically modified silica gel, wherein zwitterions are dissolved in the buffered solvents, or the pH of the solvent mixture is in the vicinity of the isoelectric point of the insulin or insulin derivative to be purified and zwitterions are present.

2. The process as claimed in claim 1, wherein the pH of the solvent mixture is up to 1 pH unit below or above the isoelectric point of the insulin or insulin derivative to be purified.

3. The process as claimed in claim 2, wherein the pH of the solvent mixture is up to 0.5 pH units below or above the isoelectric point.

4. The process as claimed in one or more of claims 1 to 3, wherein α-amino acids or betaines are employed as zwitterions.

5. The process as claimed in claim 4, wherein glycine, glutamic acid or glycine betaine are employed as zwitterion.

11

6. The process as claimed in one or more of claims 1 to 5, wherein insulin derivatives of the formula I

in which

R$^{30}$ is the residue of a genetically encodable L-amino acid,

X is a hydroxyl group, a physiologically acceptable organic group which is basic in nature and has up to 50 carbon atoms, a genetically encodable L-amino acid whose terminal carboxyl functionality which is present where appropriate can be free, as ester functionality, as amide functionality, as lactone or reduced to $CH_2OH$,

n is an integer from 0 to 10,

Y is hydrogen or L-phenylalanine, and the A and B chains are the sequences of animal or human insulin,

are used.

7. The process as claimed in claim 6, wherein in formula I
R$^{30}$ is L-alanine or L-threonine and X is one or more L-amino acids from the group comprising L-arginine, L-lysine or L-phenylalanine.

8. The process as claimed in one or more of claims 1 to 7, wherein a preparative high-pressure liquid chromatography system is employed.

## Revendications

1. Procédé pour la purification de l'insuline et/ou de dérivés d'insuline par chromatographie dans des solvants aqueux tamponnés, qui contiennent des solvants organiques miscibles à l'eau, sur un gel de silice à modification lipophile, caractérisé en ce que des zwitterions sont dissous dans les solvants tamponnés, ou en ce que des zwitterions sont présents et le pH du mélange de solvants se situe au voisinage du point isoélectrique de l'insuline ou du dérivé d'insuline à purifier.

2. Procédé selon la revendication 1, caractérisé en ce que le pH du mélange de solvants peut aller jusqu'à 1 unité de pH au-dessous ou au-dessus du point isoélectrique de l'insuline ou du dérivé d'insuline à purifier.

3. Procédé selon la revendication 2, caractérisé en ce que le pH du mélange de solvants peut aller jusqu'à 0,5 unité de pH au-dessous ou au-dessus du point isoélectrique.

4. Procédé selon une ou plusieurs des revendications 1 à 3, caractérisé en ce que, en tant que zwiterion on utilise des α-aminoacides ou des bétaïnes.

5. Procédé selon la revendication 4, caractérisé en ce que, en tant que zwitterion on utilise la glycine, l'acide glutamique ou la glycine-bétaïne.

6. Procédé selon une ou plusieurs des revendications 1 à 5, caractérisé en ce que l'on utilise des dérivés d'insuline de formule I

(I)

dans laquelle

$R^{30}$ représente le reste d'un L-aminoacide génétiquement codable,

X représente un groupe hydroxy, un groupe organique physiologiquement acceptable, à caractère basique, ayant jusqu'à 50 atomes de carbone, un L-aminoacide génétiquement codable et dont la fonction carboxy terminale, éventuellement présente, peut se trouver sous forme libre, sous forme de fonction ester, sous forme de fonction amide, sous forme de lactone ou réduite en $CH_2OH$,

n représente un nombre entier allant de 0 à 10,

Y représente un atome d'hydrogène ou un résidu L-phénylalanine, et les chaînes A et B sont les séquences de l'insuline humaine ou d'une insuline animale.

7. Procédé selon la revendication 6, caractérisé en ce que, dans la formule I,

$R^{30}$ représente un résidu L-alanine ou L-thréonine et

X représente un ou plusieurs L-aminoacides choisis parmi la L-arginine, la L-lysine ou la L-phénylalanine.

8. Procédé selon une ou plusieurs des revendications 1 à 7, caractérisé en ce que l'on utilise une unité de chromatographie liquide à haute pression préparative.

13